# EUROPEAN PATENT APPLICATION

(11) **EP 1 604 693 A1**
(43) Date of publication of application: **14.12.2005**
(21) Application number: 04013668.1
(22) Date of filing: 09.06.2004
(51) Int. Cl.: A61L 27/34, A61L 27/54, A61L 27/12, A61L 27/56, A61L 27/58

(54) **In situ forming scaffold, its manufacturing and use**

(71) Applicant: Scil Technology GmbH, 82152 Martinsried (DE)
(72) Inventor: Schütz, Andreas, 82152 Krailling (DE); Siedler, Michael, 80538 München (DE); Hellerbrand, Klaus, 82269 Geltendorf (DE)
(74) Representative: Grünecker, Kinkeldey, Stockmair & Schwanhäusser Anwaltssozietät

(57) **Abstract**

An in-situ-hardening paste, containing an organic or inorganic filling material, a biodegradable polymer and a water soluble polymeric plasticizer was developed as a delivery system for an active agent with scaffold properties in the filed of tissue regeneration. The hardened paste is sufficient mechanical stable to can be used as bone and cartilage replacement matrix. All components are full biocompatible, preferably bioresorbable and certified for parenteral application. The sustained release of peptides and proteins can be modulated by the composition and process design.

The invention encompasses a pharmaceutical composition comprising the paste of the invention and relates to the use of said paste for the preparation of a pharmaceutical composition to be used for bone augmentation, for treating bone defects, for treating degenerative and traumatic disc disease, for treating bone dehiscence or to be used for sinus floor elevation. Finally, the invention relates to a kit comprising the paste of the invention.

## Description

An in-situ-hardening paste, containing an organic or inorganic filling material, a biodegradable polymer and a water soluble polymeric plasticizer was developed as a delivery system for an active agent with scaffold properties in the filed of tissue regeneration. The hardened paste is sufficient mechanical stable to can be used as bone and cartilage replacement matrix. All components are full biocompatible, preferably bioresorbable and certified for parenteral application. The sustained release of peptides and proteins can be modulated by the composition and process design.

The invention encompasses a pharmaceutical composition comprising the paste of the invention and relates to the use of said paste for the preparation of a pharmaceutical composition to be used for bone augmentation, for treating bone defects, for treating degenerative and traumatic disc disease, for treating bone dehiscence or to be used for sinus floor elevation. Finally, the invention relates to a kit comprising the paste of the invention.

### Background Technology

Various calcium phosphates such as beta-tricalcium phosphate (Ca₃(PO₄)₂) (beta-TCP), alpha-tricalcium phosphate (alpha-TCP) and hydroxyapatite (HA) have been shown to be effective as bone replacement materials. Beta-TCP, for example, is suitable both as granulate and in pieces (blocks) for the treatment of bone defects. The bone replacements materials containing calcium phosphate are usually used when the regeneration of the bone is not possible any more or is possible with difficulties only. In addition, bone replacement materials are used when the formation of additional bone is a prerequisite for a subsequent setting of an implant. The calcium phosphates exhibit an osteoconductive effect, i.e. they represent an inert structure facilitating the migration of cells from the neighbouring bone. The presence of bones or different mesenchymal cells, however, is a precondition for the new formation of bones.

The effect of calcium phosphates can be significantly increased by adding bone chips. The bones are not only osteoconductive, but also osteoinductive, i.e. they cause the transformation of undifferentiated mesenchymal stem cells into osteoblasts and chondrocytes. For reasons of safety, autogenic bone chips are preferred to the allogenic or xenogenic preparations. The production of autogenic bones, however, always involves a second surgical procedure, which, in many cases, is not accepted by the patient.

Another important biomaterial class which plays a predominant role in the field of bone tissue engineering are bioresorbable polymers (Vert, 1989). Especially the compound class of poly(hydroxy acids) has interesting application prospects due to their intrinsic biodegradability. These materials of which poly(glycolic acid) (PGA) and poly(lactic acid) (PLA) are the most prominent undergo hydrolytic chain cleavage (degradation) in a moist environment. Sustained degradation finally leads to the corresponding hydroxy acid units. Most of these hydrolytic end products occur as metabolites of many bacteria and cell phenotypes.

The degradation potential and their mechanical properties offers application for the use as substrates for temporary implants in medical technology. Studies for various polymers in different tissues document the excellent biocompatibility of these compounds in vivo forming the bases for the development of commercial implants as medical devices (Middleton, 2000).

In clinical surgery, polyesters presumably play the most important role in connection with the fixation, augmentation and replacement of bone. Devices like screws, plates, anchors or pins serve for positioning and fixation of bone fragments after bone loss or damage. The major feature of these absorbable polymers in application is the lacking necessity for a removal operation. Another important point is in favor of polymeric fixation devices: the mechanical integration of the implant in the bone tissue.

An important drawback in totally polyester based applications is the possible accumulation of degradation products reaching cytotoxic levels and the accompanying acidification at the implant site due to the pH lowering release of acid monomers.

To avoid such negative consequences caused by local pH decrease it has been suggested to incorporate basic salts within PLA / PGA implants (Agrawal et al., 1997). Other approaches employ basic ceramics like calcium phosphates as fillers for polyesters to balance the local pH value and increase the mechanical stability (Schiller 2003). Also composites of hydroxyapatite and PLA/PLGA (polylactide-co-glycolide) have been produced (Durucan 2000). The processing of such composites requires thermal treatment and the use of chloroform solutions of the polymeric component. As an alternative one can use polymers with higher hydrolytic stability like poly(hydroxybutyric acid).

For the use of these polymers as bone substitutes the common strategy is to design a implant which temporarily fulfills the function to allow a healing process and to retain strength during the early stages at the implantation site after operation. Afterwards the loss of strength and modulus of the implant should be in harmony with the increasing strength of the injured tissue (Tormälä, 1995). Proceeding degradation creates space for restoring processes to fill the gap with ingrown of vital host tissue. Presently, no filling material is available that fits this requirements satisfactorily to form new homogeneous bone in large defects (Rueger 1996).

To achieve an osteoinductive effect an alternative to the use of autogenic bones is the use of specific bone growth and differentiation factors such as GDF-5 or different bone morphogenetic proteins (BMPs). The osteoinductive effect, however, can only be exerted by these protein factors if they are used in an immobilized form. In the literature, calcium phosphates, collagen, mineralised collagen (collagen-containing calcium phosphate) and bioresorbable polymers are described as carriers (hydroxy apatite and beta-TCP (Hotz, 1994), hydroxylic apatite from algae extracts (Gao, 1996), bone extracts (Gombotz, 1996), collagen (Friess, 1999) and poly(α-hydroxy acids) (Hollinger, 1996).

The analyses of the potency of the coated carriers, which are described in the literature, do not present a uniform picture but exhibit significant variations which are a consequence of either the carrier type selected or the coating method (Terheyden et al. (1997)). Various methods are described.

In WO 98/21972 coating by rapid precipitation of GDF-5 onto beta-TCP is achieved by first dissolving GDF-5 in an organic solvent and then precipitating it by adding water. Due to the toxicity of many solvents, however, such a process is not preferred for the production of pharmaceutical compositions. Lind et al. (1996) carry out the coating of various calcium phosphate ceramics in the presence of gelatine (usually obtained from bovine or pig bones) as protection protein. Due to the increased risk of infection and immunogenic reactions, however, the use of animal substances should be avoided for the production of pharmaceutical compositions and medicinal products. Friess et al. (1999) and Gao et al. (1996) describe the coating of collagens with BMP-2. Due to the low compressive strength of collagens, such carriers, however, are not suitable for many indications. This particularly applies to indications with which the newly-formed bone has to sustain a later pressure load. Furthermore, pharmaceutical qualities of collagen are so far available from animal sources only. Finally, according to the fast degradation rate and release of the growth factors in the state of the art products (e.g. rhBMP-2 and collagen sponge) the drug substance content is often dramaticaly above the physiological level in the bone tissue.

Advantageously, as disclosed in WO 03/043673, it has been found by the present inventors that improved and reliable osteoinductive and osteoconductive properties in vivo after implantation into a subject, preferably a human, is achieved in a device, wherein a homogenous distribution of the composite carrier, such as β-TCP or other calcium phosphates, with biologically active, non-aggregated osteoinductive protein can be realized. Such aggregation causes micro-precipitation, which is the reason for an inhomogenous distribution resulting in at least significantly decreased osteoinductive properties as described for other devices in the prior art, e.g., in WO98/21972. Moreover, it has been found that undesirable side effects, such as inflammation and toxic reactions of the subject after implantation, can be avoided by the device of the present invention which is free of toxic impurities or infectious contaminants. In particular, the use of protecting proteins (such as e.g. gelatine) as solubility mediator is totally unnecessary for the device of WO 03/043673. However, such devices are not suitable for applications requiring a retarded release of the active agent.

In the field of bone augmentation retarded release systems are especially required in view of short half-life of proteins or peptides in the human body with respect to bone induction, either due to dispersion from the implant site or through degradation. In first attempts to achieve a retarded release of bone morphogenic proteins, devices have been disclosed, wherein such proteins have been combined with bioresorbable polymers. Hollinger et al (1996) published the use of poly (α-hydroxy acids) as carriers for BMP-2. In combination with osteogenic proteins or peptides these polymers are of special interests with regards to achieve a controlled release of the active agent. Whang et al (2000) disclose an emulsion freeze-drying process starting with a PLA solution in methylene chloride for the fabrication of a biodegradable scaffold capable of incorporating and delivering bioactive macromelecules for bone regeneration. Schmidmaier et al (2000) disclose the use of a chloroform solution of PLA together with the osteoinductive factors IGF-I and TGF-□ 1 in the coating implants.

WO02/070029 discloses a porous β-TCP matrix which is optionally admixed with PLGA microspheres encapsulated with OP-1 (osteogenic protein 1, a bone morphogenic protein) to form a heterogeneous material. In contrast to WO 03/043673 the β-TCP matrix in WO02/070029 exhibits single separate voids instead of interconnected pores. The pores of this matrix are not capable to be equipped with a homogeneous coating of the polymer and/or active agent component. The microspheres are produced by Alkmeres, Inc and exhibit a 20 to 500 µm diameter permitting microaggregation of the encapsulated active agent. For the production of such microspheres methylene chloride solutions of the polymeric component together with the protein are sprayed and frozen in a deeply cold ethanol. (Herbert, P. et al. 1998 and see e.g. US Patent Application No 6,726,860), both steps in combination with two different organic solvents imparting chemical and mechanical stress to the protein.

Placing medical devices such as implants and other solid articles in a body frequently involves a surgical procedure. For some applications e.g. drug delivery however, it has been described that biodegradable polymer based delivery systems can be introduced in a body as flowable formulations (see e.g. US Pat. Nos 6,461,631; 5,780,044 and 5,278,202, all of them assigned to Atrix Laboratories, Inc.). These formulations are administered using a syringe to form in situ a solid matrix. However, the subsequent *in vivo* degradation of the polymer causes the same problems as described above for conventional polymer based scaffolds.

Accordingly, an object underlying the present invention is the provision of an in situ forming paste suitable for implantation into a subject in the need of bone augmentation by injection, molding, filling or pressing. In situ the paste allows the retarded release of an active agent attached to the matrix preferably with an optimized local activity of said active agent.

Another kind of injectable system which is delivered via injection and set in situ are calcium phosphate cements. These materials were delivered into the bone defect as a moldable paste and become solid after a period of time. (Driessens F.C.M. et al. 2002) Recently these systems become interesting especially in combination with bone morphogenetic proteins. (Seeherman H. 2003). In contrast to the PLGA containing carriers, were the protein is located and protected within the polymeric matrix the protein is in direct contact with the surrounded medium. Furthermore the protein or peptide is released only by diffusion from the calcium phosphate cement whereas the protein or peptide within the polymer matrix is released with the increasing degradation of the polymer and/or by diffusion from the polymer matrix. Therefore the release kinetic can be fine-tuned more easily than it's the case for the pure calcium phosphate cement. Disadvantages by using these pure calcium phosphate cements are their low mechanical stability (e.g. brittleness) and their compact structure.

Another object underlying the present invention is the provision of an in situ forming paste suitable for implantation into a subject in the need of bone augmentation by injection allowing retarded release of an attached active agent and avoiding the problems associated with a local pH decrease induced by polymer degradation.

Another object underlying the present invention is the provision of an in situ forming paste suitable for implantation into a subject in the need of bone augmentation by injection allowing retarded release of an attached active agent and avoiding toxic side effects and / or inflammatory responses.

Another object underlying the present invention is the provision of an in situ forming paste suitable for implantation into a subject in the need of bone augmentation by injection allowing retarded release of an attached active agent and allowing lower doses of the active agent compared to conventional devices.

Another object underlying the present invention is the provision of an in situ forming paste suitable for implantation into a subject in the need of bone allowing retarded release of an attached active agent by injection which paste is capable of forming a mechanically stable and porous scaffold for bone and carilage defect filling.

Another object underlying the present invention is the provision of an in situ forming paste suitable for implantation into a subject in the need of bone allowing retarded release of an attached active agent by injection which paste hardenes under physiological conditions and is capable of replacing conventional devices implanted via surgery.

### Summary of the Invention

Surprisingly, the present inventors were able to provide an in situ forming paste solving these objects and corresponding methods for the production of said paste.

Accordingly, the present invention provides a method for the production of an in situ forming paste comprising the steps of:
(a) providing solution comprising a plasticizer, which is a water soluble polymer, and a water insoluble polymer to get a viscous liquid;
(b) dissolving an active agent in said viscous liquid; and
(c) mixing said viscous liquid consisting of the water insoluble polymer and the plasticizer with the water insoluble solid filler to prepare a paste

This first method ("method A") is suitable for an active agent soluble the above viscous liquid.

Additionally, a method for the production of an in situ forming paste comprising the steps of:
(a) providing solution comprising a plasticizer, which is a water soluble polymer, and a water insoluble polymer to get a viscous liquid; and
(b) mixing said viscous liquid consisting of the water insoluble polymer and the plasticizer with a water insoluble solid filler to prepare a paste, wherein said filler comprises an active agent which is coated on said solid filler
is provided.

This second method ("method B") is suitable also for an active agent insoluble in the above viscous liquid.

The present invention further relates to a paste produced by one of said methods, wherein said methods optionally comprise the step of adding a water soluble filler for pore building in said paste.

The present invention further relates to a paste produced by one of said methods, which paste has osteoinductive and osteoconductive properties in vivo, comprises a carrier, wherein an active agent, preferably an osteoinductive protein, is coated onto the carrier together with a polymer, preferably a biodegradable polymer.

### Brief description of the Figures

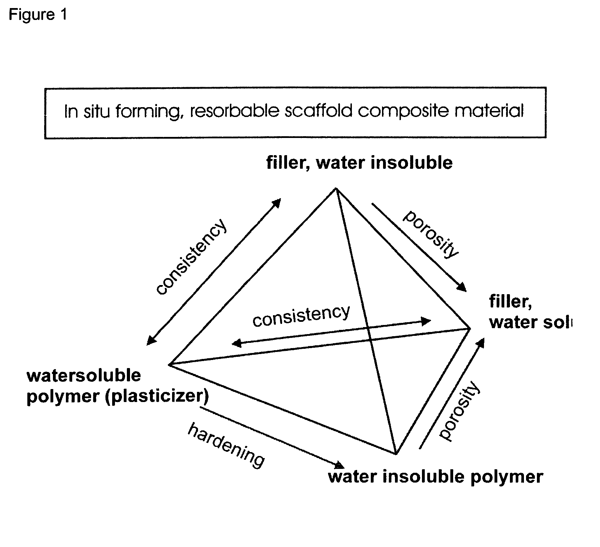

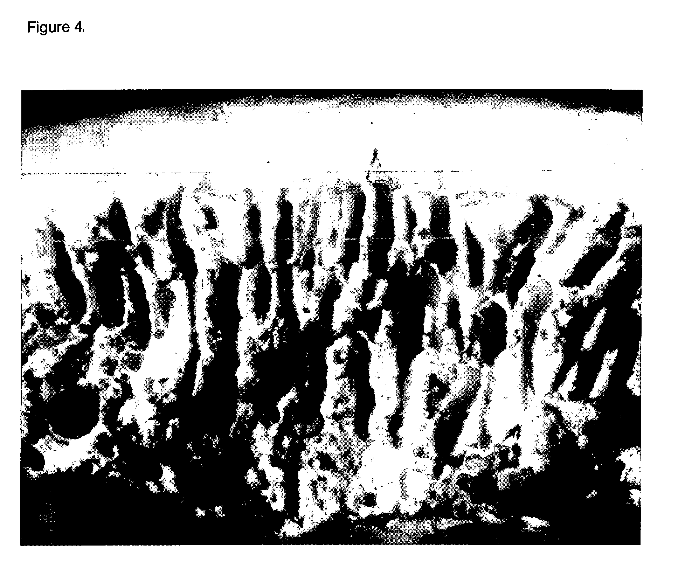

Figure 1: Scheme
   Shows the influence of the ingredients of the in situ forming paste referring to its consistency, porosity and hardening characteristics depending on their ratio.
Figure 2: Preparation of the test samples in a hollow mould
   Shows the solid form-giving device that is used to prepare the test samples of defined shape. The paste ist filled into the device and incubated in aqueous medium to mimic and monitor and analyse the in situ hardening process. Subsequently the samples can be used for analytical testing
Figure 3: Location of the cross sections trough the test sample
   Shows the site of the cross sections through the test samples displayed in figures 4 and 5.
Figure 4: axial cut through the cylindrical shaped test sample (40 % PLGA, 60 % TCP 5 x 10 mm), shows tube like pores
Figure 5: radial cut through the cylindrical shaped test sample (40 % PLGA, 60 % TCP 5 x 10 mm) shows tube like pores
Figure 6: fracture through the cylindrical shaped test sample (40 % PLGA, 60 % TCP 5 x 10 mm) shows tube like pores

### Detailed description of the preferred embodiments

The term "paste" as used in accordance to the present invention refers to an paste like entity administerable using a syringe, which comprises at least three components as set forth below. The paste is preferably suitable for surgical defect filling and tissue regeneration. In another embodiment the paste is a drug delivery system for the controlled release of active substances after implantation.

One of the components of said paste is the so-called "water insoluble filler". This called "water insoluble filler" serves as matrix for the paste, and as carrier for the scaffold formed in *vivo*, once the paste is hardened. Preferably said said water insoluble solid filler is selected from calcium phosphate, chitosan, collagen, alginate, calcium sulfate, cacium carbonate, magnesiumoxide, magnesium hydroxide, magnesium carbonate, silicium dioxide, poly(2-hydroxyethyl methacrylate), hyaluronic acid and derivatives, cellulose and derivatives, starch and derivatives and / or any combinations thereof.

Preferably, said "water insoluble filler" forms an inorganic matrix, which consists of ceramics, serving in the hardened state as "osteoconductive carrier". Preferably, said inorganic matrix consists of ceramics. More preferably, said carrier is a calcium phosphate. Most preferably said inorganic matrix is a calcium phosphate, which is beta tricalcium phosphate, alpha tricalcium phosphate, apatite or a calcium phosphate containing cement. Alternatively, said carrier is selected from the group consisting of calcium carbonate, magnesium carbonate, magnesium oxide or magnesium hydroxide.

More preferably, said inorganic matrix is a calcium phosphate. Most preferably said inorganic matrix is a calcium phosphate, which is beta tricalcium phosphate, alpha tricalcium phosphate, apatite or a calcium phosphate containing cement. Alternatively, said carrier is preferably selected from the group consisting of calcium carbonate, magnesium carbonate, magnesium oxide or magnesium hydroxide.

Said ceramics may have a particularly high surface due to the small particles size or the presence of macro- and micropores. In a preferred embodiment, said macropores have a diameter of 100 to 400 µm. In another preferred embodiment, said micropores have a diameter of less than 10 µm. In still another preferred embodiment, the pores are interconnected to allow the influx of coating substances, e.g. in the preparation of a device consisting of said ceramics and an active agent, as well as in-growth of bone and tissue cells in the application in vivo.

The term "carrier" encompasses three-dimensional matrices, such as the above-mentioned ceramics and ceramic/polymer composites. The carrier, preferably, has an enlarged surface due to the small particles size or the formation of macro- and micro- pores during the manufacturing process.

The term "calcium phosphate" encompasses compositions comprising calcium ions (Ca²⁺), phosphate ions (PO₃³⁻), optionally, further ions like hydroxyl ions (OH⁻), carbonate (CO₃²⁻) or magnesium (Mg²⁺) or other ions which are suitable for the carrier of the present invention. The calcium phosphates as used in accordance with the present invention are crystals having a three dimensional structure suitable for the paste of the present invention as set forth above. Said calcium phosphates are particularly well suited as carriers for the paste of the present invention. Their in vivo properties have been described in Hotz, 1994, Gao, 1996, and in WO98/21972. A list of preferred and well-known calcium phosphates is given above.

The second component of the paste of the present invention is a water insoluble polymer. Preferably said water insoluble polymer is a "biocompatible", a "biodegradable" or a "bioresorbable" polymer.

The term "biocompatible" means the ability of a material to perform with an appropriate host response in a specific application (Wintermantel E. et al.). "Biodegradable polymers" specifies polymers, which break down due to macromolecular degradation with dispersion in vivo but for which no proof exists for the elimination from the body. The term "bioresorbable polymer" specifies polymeric materials, which underwent degradation and further resorption in vivo; i.e. polymers, which are eliminated through natural pathways either because of simple filtration of degradation by-products or after their metabolization. Bioresorption is thus a concept which reflects total elimination of the initial foreign material.

In a preferred embodiment of the paste or the method of the invention said bioresorbable polymer is a polymer that undergoes a chain cleavage due to macromolecular degradation in an aqueous environment.

Alternatively, said polymer is selected from the group consisting of polyethylene (PE), polypropylene (PP), polyethylenerephthalate (PET), polyglactine, polyamide (PA), polymethylmethacrylate (PMMA), polyhydroxymethylmethacrylate (PHEMA), polyvinylchloride (PVC), polyvinylalcohole (PVA), polytetrafluorethylene (PTFE), polyetheretherketone (PEEK), polysulfon (PSU), polyvinylpyrolidone, polyurethane or polysiloxane. These polymers are at least biocompatible.

More preferably, said polymer is selected from the group consisting of poly(α-hydroxy acids), poly (ortho esters), poly(anhydrides), poly(aminoacids), polyglycolid (PGA), polylactid (PLLA), poly(D,L-lactide) (PDLLA), poly(D,L-lactide co-glycolide) PLGA), poly(3-hydroxybutyricacid) (P(3-HB)), poly(3-hydroxy valeric acid) (P(3-HV)), poly(p-dioxanone) (PDS), poly(ε-caprolactone) (PCL), polyanhydride (PA). These polymers are biocompatible and bioresorbable.

The third component of the paste of the present invention is an "active agent". The term "active agent" comprises a polypeptide or a small molecule drug which is immobilized on and/or in the carrier. Preferably, said polypeptide or drug is homogeneously distributed on the calcium phosphate containing carrier and / or within the polymer.

The term "osteoconductive" refers to substrates that provide a favourable scaffolding for vascular ingress cellular infiltration and attachment, cartilage formation, and calcified tissue deposition. Osteoconductive materials may support osseous generation via the scaffolding effect (Kenley, R.A., 1993)

The term "osteoinductive" refers to the capability of the transformation of mesenchymal stem cells into osteoblasts and chondrocytes. A prerequisite for osteoinduction is a signal which is distributed by the paste into the surrounding tissues where the aforementioned osteoblast precursors become activated. Osteoinduction as used herein encompasses the differentiation of mesenchymal cells into the bone precursor cells, the osteblasts. Moreover, osteoinduction also comprises the differentiation of said osteoblasts into osteocytes, the mature cells of the bone. Moreover, also encompassed by osteoinduction is the differentiation of mesenchymal cells into chondrocytes. In particular in the long bones, the chondroblasts and the chondrocytes residing in the perichondrium of the bone can also differentiate into osteocytes. Thus, osteoinduction requires differentiation of undifferentiated or less-differentiated cells into osteocytes which are capable of forming the bone. Thus, a prerequisite for osteoinduction is a signal which is distributed by the paste into the surrounding tissues where the aforementioned osteocyte precursors usually reside. As has been described above, the osteoinductive proteins or peptides used in accordance with the present invention are sustained released from the paste, once said paste has in situ formed a hard scaffold after implantation and said osteoinductive proteins or peptides are subsequently distributed efficiently in the surrounding tissues.

Moreover, the proteins and peptides encompassed by the present invention have osteoinductive properties in vivo. For example, it is well known in the art that the Transforming Growth Factor-β (TGF-β) superfamily encompasses members which have osteoinductive properties. Individual members of said TGF-β superfamily which have particular well osteoinductive properties are listed infra. In conclusion, the osteoinductive proteins or peptides of the paste of the present invention after having been released from the carrier serving as a osteoinductive signal for the osteocyte precursors of the tissue surrounding the side of implantation of the paste.

The term "osteogenic" describes the synthesis of new bone by osteoblasts. In accordance with the present invention, preexisting bone in the surrounding of the side of implantation of the paste grows into the hardened paste using the structure of the hardened paste, especially formed during the hardening process, as a matrix onto which the osteocytes can adhere).

The term "osteoinductive polypeptide" refers to polypeptides, such as the members of the Transforming Growth Factor-β (TGF-β) superfamily, which have osteoinductive properties.

In a further preferred embodiment of the paste or the method of the invention said osteoinductive protein is a member of the TGF-β family.

The TGF-β family of growth and differentiation factors has been shown to be involved in numerous biological processes comprising bone formation. All members of said family are secreted polypeptides comprising a characteristic domain structure. On the very N-terminus, the TGF-β family members comprise a signal peptide or secretion leader. This sequence is followed at the C-terminus by the prodomain and by the sequence of the mature polypeptide. The sequence of the mature polypeptide comprises seven conserved cysteins, six of which are required for the formation of intramolecular disulfide bonds whereas one is required for dimerization of two polypeptides. The biologically active TGF-β family member is a dimer, preferably composed of two mature polypeptides. The TGF-β family members are usually secreted as proteins comprising in addition to the mature sequence the prodomain. The prodomains are extracellularly cleaved off and are not part of the signalling molecule. It has been reported, however, that the prodomain(s) may be required for extracellular stabilization of the mature polypeptides.

In the context of the present invention, the term "TGF-β family member" or the proteins of said family referred to below encompass all biologically active variants of the said proteins or members and all variants as well as their inactive precursors. Thus, proteins comprising merely the mature sequence as well as proteins comprising the mature protein and the prodomain or the mature protein, the prodomain and the leader sequence are within the scope of the invention as well as biologically active fragments thereof. Whether a fragment of a TGF-β member has the biological activity can be easily determined by biological assays described, e.g. in: Katagiri T, Yamaguchi A, Ikeda T, Yoshiki S, Wozney JM, Rosen V, Wang EA, Tanka H, Omura S, Suda T, (1990): The non-osteogenic mouse pluripotent cell line, C3H10T1/2, is induced to differentiate into osteoblastic cells by recombinant human bone morphogenetic protein-2. Biochem. Biophys. Res. Commun. 172: 295-299 or Nishitoh H, Ichijo H, Kimura M, Matsumoto T, Makishima F, Yamaguchi A, Yamashita H, Enomoto S, Miyazono K (1996): Identification of type ( and type (( serine/threonine kinase receptors for growth/ differentiation factor-5. J. Biol. Chem. 271: 21345-21352.

Preferably, the biological activity according to the invention can be determined by in vivo models as described in the accompanied Examples. Furthermore, encompassed by the present invention are variants of the TGF-β members which have an amino acid sequences being at least 75%, at least 80%, at least 90%, at least 95%, at least 96%, at least 97%, at least 98% or at least 99% identical to the amino acid sequences of the members of the TGF-β family.

An overview of the members of the TGF-β superfamily is given in: Wozney JM, Rosen V (1998): Bone morphogenetic protein and bone morphogenetic protein gene family in bone formation and repair. Clin Orthop 346: 26-37. The amino acid sequences of the members of the TGF-β family can be obtained from the well known databases such as Swiss-Prot via the internet (http://www.expasy.ch/sprot/sprot-top.html) Amino acid sequences for BMP2, BMP7 and GDF-5, members of the TGF-( family with a particularly high osteoinductive potential, are also shown in SEQ ID No: 1 to 3, respectively. Amino acid sequences for BMP2, BMP7 and GDF-5, members of the TGF-β family with a particularly high osteogenic potential, are also shown in SEQ ID No:1 to 3, respectively.

More preferably, said member of the TGF-β family is a member of the BMP subfamily. The members of the Bone Morphogenetic Protein (BMP) subfamily have been shown to be involved, inter alia, in the induction and re-modeling of bone tissue. BMPs were originally isolated from bone matrix. These proteins are characterized by their ability to induce new bone formation at ectopic sites. Various in vivo studies demonstrated the promotion of osteogenesis and chondrogenesis of precursor cells by BMPs and raise the possibility that each BMP molecule has distinct role during the skeletal development. More details about the molecular and biological properties of the BMPs are described in:
Wozney JM, Rosen V (1998): Bone morphogenetic protein and bone morphogenetic protein gene family in bone formation and repair. Clin Orthop 346: 26-27, Schmitt J, Hwang K, Winn, SR, Hollinger J (1999): Bone morphogenetic proteins: an update on basic biology and clinical relevance. J Orthop Res 17: 269-278 and Lind M (1996): Growth factors: possible new clinical tools. A review. Acta Orthop Scand 67: 407-17.

The osteoinductive polypeptide of the present invention is preferably selected from the group consisting of BMP-1, BMP2, BMP-3, BMP-4, BMP-5, BMP-6, BMP7, BMP-8, BMP-9, BMP-10, BMP-11, BMP-12, BMP-13, BMP-14, BMP-15 and BMP-16. Most preferably, said member of the BMP family is BMP2 or BMP7.

The amino acid sequence for the preproform of BMP2 is deposited under Swiss-Prot Accession number P12643 and is shown below. Amino acids 1 to 23 correspond to the signal sequence, amino acids 24 to 282 correspond to the propeptide and amino acids 283 to 396 correspond to the mature protein. The amino acid sequence for the preproform of BMP7 is deposited under Swiss-Prot Accession number P18075 or shown in SEQ ID No: 2. Amino acids 1 to 29 correspond to the leader sequence, amino acids 30 to 292 correspond to the proform and amino acids 293 to 431 correspond to the mature protein. Preferably, BMP-2 or BMP7 refers to the preproform, to the proform or to the mature BMP-2 or BMP-7 peptide, respectively. Moreover also encompassed are fragments of said proteins having essentially the same biological activity, preferably osteoinductive properties. More sequence information for BMP2 and BMP7 is provided below.

Alternatively, the osteoinductive polypeptide of the present invention is selected from another TGF-β family, i.e. the GDF family.

Growth and Differentiation Factor (GDF) have been also shown to be involved, inter alia, in the induction and re-modeling of bone tissue. Growth Differentiation Factor 5 (GDF-5), also known as cartilage-derived morphogenetic protein 1 (CDMP-1) is a member of subgroup of the BMP family, which also includes other related proteins, preferably, GDF-6 and GDF-7. The mature form of the protein is a 27 kDa homodimer. Various in vivo and in vitro studies demonstrate the role of GDP-5 during the formation of different morphological features in the mammalian skeleton. Mutations of GDF-5 are responsible for skeletal abnormalities including decrease of the length of long bones of limbs, abnormal joint development in the limb and sternum (Storm & Kingsley (1999), Development Biology, 209, 11-27). The amino acid sequence between mouse and human is highly conserved.

Preferably, the osteoinductive polypeptide of the present invention is selected from the group consisting of GDF-1, GDF-2, GDF-3, GDF-4, GDF-5, GDF-6, GDF-7, GDF-8, GDF-9, GDF-10 and GDF-11. Most preferably, said member of the GDF subfamily is GDF-5.
The amino acid sequence for the preproform of GDF-5 is deposited under Swiss-Prot Accession number P 43026 or shown in SEQ ID No: 3. Amino acids 1 to 27 correspond to the leader sequence, amino acids 28 to 381 correspond to the proform and amino acids 382 to 501 correspond to the mature protein. Preferably, GDF-5 refers to the preproform, to the proform or to the mature GDF-5 peptide. Moreover also encompassed are fragments of GDF-5 having essentially the same biological activity, prefrably osteoinductive properties. Most preferably, said fragment comprises amino acids 383 to 501 of the sequence shown in SEQ ID No: 3.

The following tables show amino acid sequences for BMP-2, BMP-7 and GDF-5:

Human BMP-2 (Swiss-Prot Prim. Accession Number P12643); SEQ ID No. 1:

Key
From To
Length

SIGNAL
1 23
23

PROPEP
24 282
259

hBMP2
283 396
114

10 20 30 40 50 60
| | | | | |
MVAGTRCLLA LLLPQVLLGG AAGLVPELGR RKFAAASSGR PSSQPSDEVL SEFELRLLSM

70 80 90 100 110 120
| | | | | |
FGLKQRPTPS RDAVVPPYML DLYRRHSGQP GSPAPDHRLE RAASRANTVR SFHHEESLEE

130 140 150 160 170 180
| | | | | |
LPETSGKTTR RFFFNLSSIP TEEFITSAEL QVFREQMQDA LGNNSSFHHR INIYEIIKPA

190 200 210 220 230 240
| | | | | |
TANSKFPVTR LLDTRLVNQN ASRWESFDVT PAVMRWTAQG HANHGFVVEV AHLEEKQGVS

250 260 270 280 290 300
| | | | | |
KRHVRISRSL HQDEHSWSQI RPLLVTFGHD GKGHPLHKRE KRQAKHKQRK RLKSSCKRHP

310 320 330 340 350 360
| | | | | |
LYVDFSDVGW NDWIVAPPGY HAFYCHGECP FPLADHLNST NHAIVQTLVN SVNSKIPKAC

370 380 390
| | |
CVPTELSAIS MLYLDENEKV VLKNYQDMVV EGCGCR

### References

[1]
   SEQUENCE FROM NUCLEIC ACID.
   MEDLINE=89072730; PubMed=3201241;
   Wozney J.M., Rosen V., Celeste A.J., Mitsock L.M., Whitters M.J., Kriz R.W., Hewick R.M., Wang E.A.;
   "Novel regulators of bone formation: molecular clones and activities."; Science 242:1528-1534(1988).
[2]
   X-RAY CRYSTALLOGRAPHY (2.7 ANGSTROMS) OF 292-396.
   MEDLINE=99175323; PubMed=10074410;
   Scheufler C., Sebald W., Huelsmeyer M.;
   "Crystal structure of human bone morphogenetic protein-2 at 2.7 A resolution.";
   J. Mol. Biol. 287:103-115(1999).

Human BMP-7(Swiss-Prot Prim. Accession. Number: P18075); SEQ ID No. 2:

Key
From To
Length

SIGNAL
1 29
29

PROPEP
30 292
263

hBMP-7
293 431
139

10 20 30 40 50 60
| | | | | |
MHVRSLRAAA PHSFVALWAP LFLLRSALAD FSLDNEVHSS FIHRRLRSQE RREMQREILS

70 80 90 100 110 120
| | | | | |
ILGLPHRPRP HLQGKHNSAP MFMLDLYNAM AVEEGGGPGG QGFSYPYKAV FSTQGPPLAS

130 140 150 160 170 180
| | | | | |
LQDSHFLTDA DMVMSFVNLV EHDKEFFHPR YHHREFRFDL SKIPEGEAVT AAEFRIYKDY

190 200 210 220 230 240

IRERFDNETF RISVYQVLQE HLGRESDLFL LDSRTLWASE EGWLVFDITA TSNHWVVNPR

250 260 270 280 290 300

HNLGLQLSVE TLDGQSINPK LAGLIGRHGP QNKQPFMVAF FKATEVHFRS IRSTGSKQRS

310 320 330 340 350 360

QNRSKTPKNQ EALRMANVAE NSSSDQRQAC KKHELYVSFR DLGWQDWIIA PEGYAAYYCE

370 380 390 400 410 420

GECAFPLNSY MNATNHAIVQ TLVHFINPET VPKPCCAPTQ LNAISVLYFD DSSNVILKKY

430

RNMVVRACGC H

### References

[1]
   SEQUENCE FROM NUCLEIC ACID, AND PARTIAL SEQUENCE.
   TISSUE=Placenta;
   MEDLINE=90291971; PubMed=2357959;
   Oezkaynak E., Rueger D.C., Drier E.A., Corbett C., Ridge R.J., Sampath T.K., Oppermann H.;
   "OP-1 cDNA encodes an osteogenic protein in the TGF-beta family.";
   EMBO J. 9:2085-2093(1990).
[2]
   SEQUENCE FROM NUCLEIC ACID.
   MEDLINE=91088608; PubMed=2263636;
   Celeste A.J., Iannazzi J.A., Taylor R.C., Hewick R.M., Rosen V., Wang E.A., Wozney J.M.;
   "Identification of transforming growth factor beta family members present in bone-inductive protein purified from bovine bone.";
   Proc. Natl. Acad. Sci. U.S.A. 87:9843-9847(1990).
[3]
   X-RAY CRYSTALLOGRAPHY (2.8 ANGSTROMS) OF 293-431.
   MEDLINE=96149402; PubMed=8570652;
   Griffith D.L., Keck P.C., Sampath T.K., Rueger D.C., Carlson W.D.;
   "Three-dimensional structure of recombinant human osteogenic protein 1:
   structural paradigm for the transforming growth factor beta superfamily."; Proc. Natl. Acad. Sci. U.S.A. 93:878-883(1996).

Human GDF-5 (Swiss-Prot Prim. Accession Number: P 43026); SEQ ID No. 3:

Key
From To
Length

SIGNAL
1 27
27

PROPEP
28 381
354

hGDF-5
382 501
120

10 20 30 40 50 60
| | | | | |
MRLPKLLTFL LWYLAWLDLE FICTVLGAPD LGQRPQGSRP GLAKAEAKER PPLARNVFRP

70 80 90 100 110 120
| | | | | |
GGHSYGGGAT NANARAKGGT GQTGGLTQPK KDEPKKLPPR PGGPEPKPGH PPQTRQATAR

130 140 150 160 170 180
| | | | | |
TVTPKGQLPG GKAPPKAGSV PSSFLLKKAR EPGPPREPKE PFRPPPITPH EYMLSLYRTL

190 200 210 220 230 240
| | | | | |
SDADRKGGNS SVKLEAGLAN TITSFIDKGQ DDRGPVVRKQ RYVFDISALE KDGLLGAELR

250 260 270 280 290 300
| | | | | |
ILRKKPSDTA KPAVPRSRRA AQLKLSSCPS GRQPAALLDV RSVPGLDGSG WEVFDIWKLF

310 320 330 340 350 360
| | | | | |
RNFKNSAQLC LELEAWERGR TVDLRGLGFD RAARQVHEKA LFLVFGRTKK RDLFFNEIKA

370 380 390 400 410 420
| | | | | |
RSGQDDKTVY EYLFSQRRKR RAPLATRQGK RPSKNLKARC SRKALHVNFK DMGWDDWIIA

430 440 450 460 470 480
| | | | | |
PLEYEAFHCE GLCEFPLRSH LEPTNHAVIQ TLMNSMDPES TPPTCCVPTR LSPISILFID

490 500
| |

SANNVVYKQY EDMVVESCGC R

### References

[1]
   SEQUENCE FROM NUCLEIC ACID.
   TISSUE=Placenta;
   MEDLINE=95071375; PubMed=7980526;
   Hoetten G., Neidhardt H., Jacobowsky B., Pohl J.;
   "Cloning and expression of recombinant human growth/differentiation factor 5.";
   Biochem. Biophys. Res. Commun. 204:646-652(1994).
[2]
   SEQUENCE FROM NUCLEIC ACID.
   TISSUE=Articular cartilage;
   MEDLINE=95050604; PubMed=7961761;
   Chang S., Hoang B., Thomas J.T., Vukicevic S., Luyten F.P., Ryba N.J.P., Kozak C.A., Reddi A.H., Moos M.;
   "Cartilage-derived morphogenetic proteins. New members of the transforming growth factor-beta superfamily predominantly expressed in long bones during human embryonic development.";
   J. Biol. Chem. 269:28227-28234(1994).

Also encompassed by the present invention are embodiments, wherein said active agent is selected from hormones, cytokines, growth factors, antibiotics and other natural and/or synthesized drug substances like steroids, prostaglandines etc.

Preferably, said active agent is parathyroid hormone (PTH) and/or PTH 1-34 peptide.

In another embodiment of the invention, the active agent is a "cartilage inductive" or "cartilage regenerating" protein. A preferred cartilage inductive protein is MIA/CD-RAP (MIA, melanoma inhibitory activity), more preferably human MIA/CD-RAP.

The amino acid sequence for the preproform of MIA/CD-RAP is deposited under Swiss-Prot Accession number Q 16674 or shown in SEQ ID No: 4. Amino acids 1 to 24 correspond to the signal sequence, amino acids 25 to 131 correspond to the coding sequence.

The following tables shows amino acid sequences for MIA/CD-RAP:

Human MIA/CD-RAP (Swiss-Prot Prim. Accession Number: Q 16674); SEQ ID No.
4:

Key
From | To
Length

SIGNAL
1 | 24
24

h MIA/CD-RAP
25 | 131
107

MARSLVCLGV IILLSAFSGP GVRG GPMPKL ADRKLCADQE CSHPISMAVA LQDYMAPDCR
FLTIHRGQVV YVFSKLKGRG RLFWGGSVQG DYYGDLAARL GYFPSSIVRE DQTLKPGKVD
VKTDKWDFYC Q

The forth component of the paste of the present invention is a "plasticizer". The function of the plasticizer in method A is to dissolute the active agent, the water insoluble polymer and suspands the water insoluble carrier material. In method B the function of the plasticizer ist to dissolute the insoluble polymer additionally suspending the water insoluble carrier material. In both cases during in situ hardening in contact with aqueous medium the plasticizer diffuses out of the paste, leaving pores and leading to a form stable composite device. Therefore, the plasticizer has to be a water soluble compound, a water miscible solvent or preferably a water soluble polymer. Preferably said polymer is biocompatible. More preferably, said plasticizer is selected form the group consisting of polyethylene glycol 400, PEG 200, PEG 300, PEG 600, 1,3 butandiole, castor oil, N-methyl-2-pyrrolidone, 2-pyrrolidone, C2 to C6 alkanols, propylene glycol, solketal, acetone, methyl acetate, ethyl acetate, ethyl lactate, methyl ethyl ketone, dimethylformamide, dimethyl sulfoxide, dimethyl sulfone, tetrahydrofuran, caprolactam, decylmethylsulfoxide, oleic acid, propylene carbonate, triacetin, N,N-diethyl-m-toluamide, or 1-dodecytazacycloheptan-2-one. Most preferably, said polymer is a polyethylene glycol one polypropyleneglycol.

An optional fifth component of the paste of the present invention a water soluble filler, which is added optionally in an additional step of one of the methods A or B of the present invention. The function of this component is on one hand to adapt the consistency of the composite material to the respective application on the other hand in consequence to control the porosity of the in situ hardened implant.

The variation of the concentration of the five components lead to a adaptation to a specific medical application by changes within the consistency of the injactable pase, hardening time in situ, porosity and the mechanical properties of the final implant. Additionally these variation are a potent tool in adapting the release kinetic of the active agent by changed degradation behavior of the water insoluble polymer.

In a preferred embodiment, said water soluble filler is selected from the group consisting of polyethyleneglycol 4000, sucrose, mannitol, sodium chloride, solid physiological tolerable and soluble, non toxic substances like trehalose, sorbitol physiological amino acids, e.g. glycine, glutamin, arginine, sodium citrate, sodium succinate and sodium phosphates.

The paste of the present invention may optionally, comprise additional excipients. These excipients serve the stabilization of the protein or peptide, e.g., saccharides, amino acids, polyols, detergents or maintenance of the pH, e.g., buffer substances.

In a preferred embodiment the paste of the present invention is free of toxic substances. Preferably such toxic substances are already avoided in the production process, as their production requires additional expenditure due to required removal steps during the production process and necessary expensive means for highly sensitive chemical analysis.

The term "toxic substances", in particular, encompasses those toxic organic solvents and additives which are used by the methods described in the art, which are classified by the ICH as class 2 solvents (ICH Topic Q 3 C Impurities: Residual Solvents) e.g. methylene chloride. Said substances may cause systemic or local toxic effects, inflammation and / or other reactions after implantation of devices containing said substances. Said prior art devices are therapeutically less acceptable due to said undesirable side effects, which cannot be avoided by the conventionally coating methods described in the art. Moreover, the international guidance for the development of therapeutic proteins require that in the manufacturing process harmful and toxic substances should be avoided (for details see: International Conference on Harmonization (ICH), Topic Q3C; www.emea.eu.int/). However, the paste of the present invention or a paste which is obtainable by the method of the present invention is, advantageously, free of said class 1 classified toxic substances. Moreover the present invention contains only solvents classified as class 3 by the ICH Topic Q 3C and, therefore, therapeutically well acceptable and fulfills the requirements of the regulatory authorities.

Moreover, in a further preferred embodiment of the paste or the method of the invention said paste is free of infectious material.

Besides toxic substances, infectious material comprised by a prioir art device may cause severe infections in a subject into which the device has been transplanted. Potentially infectious gelatin derived from bovine or procine bones is, however, used as a protecting protein in many state of the art methods (Lind, 1996).

Solutions sufficient for the two methods A and B of the present invention to produce the paste of the present invention are inactive towards the active agent.

The term "homogeneously distributed" means that the active agent is homogeneously distributed within the respective phase coincides to the description of method A and B above. This can be achieved by the characteristic of the active agent that either dissolves in the water soluble polymer or adsorbes to the water insoluble filler leading to a suspension of the coated water insoluble filler in the water soluble polymer. Homogenous distribution is a prerequisite for efficient release and activity of the active agent into the tissue surrounding the site of implantation. Moreover, it is to be understood that the active agent is not aggregated and partially or entirely inactivated due to precipitation or micro-precipitation, rather attachment of biologically active, non-aggregated proteins is to be achieved by homogenous coating.

The homogenous coating of the carrier with said active agent and the simultaneous and / or additional homogeneous coating with the resorbable polymer do achieve an onion-like layer structure which acts in two manners as a protective film and as diffusion barrier to slow down the dissolution of the protein or peptide to achieve a sustained release. The described methods A and B allow the homogenous distribution and immobilization of the osteoinductive active agent into and / or on the carrier and the sustained release of the active agent due to the polymeric component.

The efficacy of the coating process is, furthermore, supported by the carrier due to capillary forces resulting from the presence of numerous, preferably interconnected macro- and micropores which due to their size are capable of soaking the solutions into the pores.

Moreover, in contrast to other methods described in the art, e.g., in WO98/21972, the active agent is - according to the methods A and B of the present invention - applied by attachment to the carriers from the soluble state to achieve a homogeneous coating.

The findings underlying the present invention demonstrate that the aggregation of the proteins can be avoided in a tri-component-system by the use of suitable additives as described herein. An important precondition is the knowledge of the solubility of the osteoinductive active agent dependent on the nature of the solvent, i.e. aqueous and / or organic solvent, pH value, ionic strength and surfaces present. For method B, the active agent is homogeneously onto the carrier. This homogeneous distribution is performed as described in e.g. in WO03/043673 using an aqueous solution and a buffer. The term "aqueous solution" specifies any solution compring water. The slowing down of the pH increase caused by the contact of the coating solution with the calcium phosphates in the carrier reacting in an alkaline manner, in particular, plays an important role during the coating.

The methods A and B of the present invention, distribute the active agent homogeneously across the inner surface of the carrier material and allow binding to the surface before a precipitation of the said protein takes place. For method B, It could be demonstrated that the pH increase taking place during the coating of calcium phosphates is decelerated sufficiently by the use of a weak acid, such as acetic acid. Furthermore, the addition of organic compounds such as ethanol or sucrose proves to be additionally advantageous. Furthermore, a low ionic strength is an important precondition for successful coating of the protein or peptide onto the calcium phosphate. Moreover, our tests show that the volume of the coating solutions (solution containing active agent and / or polymer), too, has a considerable effect on the quality of both coatings.

Finally, the methods A and B of the present invention allow the use of non toxic organic solvents (see below), such as dimethyl sulfoxide, anisol or glacial acid. These solvents are routinely used in the methods described in the art. Normally they damage the protein during contacting and/or especially during drying but such damage is surprisingly avoided by using the special drying technique of the present invention, because the active agent is adsorbed/or attached onto the inorganic solid carrier.

Finally, the methods A and B of the present invention allow the use of non toxic organic solvents (see below), such as dimethyl sulfoxide, anisol or glacial acid. These solvents are routinely used in the methods described in the art. Normally they damage the protein during contacting and/or especially during drying but such damage is surprisingly avoided by using the special drying technique of the present invention, because the active agent is adsorbed/or attached onto the inorganic solid carrier.

In a preferred embodiment of the method B of the invention said buffer has a buffer concentration of 10 mmol/l to achieve a sufficient solubility of the active agent during the adsorbtion process and to avoid any modification of the monophasic calcium ceramic beta TCP. The p of the solution shifts controlled during coating and drying process from pH 3 to pH 7 T pH shift causes a reduction of the solubility of the bone growth factor homogenous, defined on the TCP.

In a further preferred embodiment of the method of the invention said buffer containing solution for method B comprises an polyol and/or alcohol. Suitable alcohols or polyols are well known in the art and are described in standard text books, such as Römpp, dictionary of chemistry. More preferably, said alcohol is ethanol and said polyol is mannitol.

In a more preferred embodiment the concentration of the polyol and or alcohol is between 0 - and 10 % (w/v).

The term "saccharides" encompasses mono-, di- and polysaccharides. The structure and composition of mono-, di-, and polysaccharides are well known in the art and are described in standard text books, such as Römpp, dictionary of chemistry.

More preferably, said saccharide is a disaccharide. Most preferably, said dissaccharide is sucrose or trehalose.

Further means and methods for controlling homogeneous distribution, quantification and characterization of the active agent are described in the accompanied examples.

Surprisingly, active agents, in particular surprisingly proteins, when adsorbed on the surface of ceramic fillers / carriers are much more resistant against degradation caused by organic solvents than proteins freely dissolved or suspended in organic solutions or integrated in biphasic emulsion systems. Thus, this aspect of the invention opens a new possibility to produce polymer based drug delivery systems for proteins without denaturation and / or modification of polypeptides in singular or multiphase organic systems.

Suitable for one of the two methods of the present invention as active agents are all proteins, polypeptides and small molecule drugs. Especially such active agents with low or no affinity for inorganic carrier matrices can be immobilized in the polymer - calcium phosphate composite material. Preferably, the binding of said active agent to the carrier is reversible.

Thereby, dissolution of said active agent is allowed once the paste has been brought into a suitable in vivo surrounding, such as a bone cavity or into the intramuscular or subcutaneous aqueous environment and hardens by diffusion out of the water soluble polymer while aqueous body fluids diffuse into the polymer. In consequence the polymer solidifies due to its insolubility in water. (Shively, A.L. et al. 1995).

More preferably, said dissolution of the immobilized compounds is a sustained release allowing diffusion of the active agent into the tissue which surrounds the hardened paste. Thus, the hardened paste is suitable to serve as an in vivo source for e.g. osteoinductive proteins, peptides or small molecule drugs, which are slowly released and which can be thereby efficiently distributed into the surrounding tissues or have an effect in the immobilized form.

The term "buffer" which assists in keeping the active agent dissolved in aqueous solutions for a time sufficient to allow "homogenous coating" refers to a component allowing the osteoinductive active agent to be efficiently dissolved and kept in the solution. This buffer is capable of avoiding and or balancing the increase of pH caused by contacting the solution with the calcium phosphate carrier so that the protein does not immediately precipitate, e.g., due to a pH increase. Said buffer can be determined by the person skilled in the art considering the solubility of the osteoinductive protein (which depends on the pH and the ionic strength) and the influence of the carrier on said parameters after contacting the carrier with said buffer containing solution. In accordance with the present invention it has been found that a suitable buffer is needed for the homogeneous distribution of the active agent onto the surface of the carrier, e.g. calcium phosphate, said buffer comprising preferably a weak acid, an alcohol and a saccharide. The solvent for the dissolution of the preferably bioresorbable polymer in which the protein or peptide is not soluble described by the method B of the present invention comprises a suitable organic solvent for the homogeneous distribution of the polymer onto the surface of the protein or peptide coated carrier e.g. dimethyl sulfoxide, anisol or glacial acid.

The invention encompasses a pharmaceutical composition comprising the paste of the invention or a paste, which is obtainable by the method of the invention.

The product of the present invention can be formulated as a pharmaceutical composition or a medical device. The composition of said product may comprise additional compounds like stabilizers, buffer substances and other excipients. The amount of the product of the present invention applied to the patient will be determined by the attending physician and other clinical factors; preferably in accordance with any of the above described methods. As it is well known in the medical arts, the amount applied to a patient depends upon many factors, including the patient's size, body surface area, age, sex, time and route of administration, general health conditions, and other drugs being administered concurrently. Progress can be monitored by periodic assessment. Thanks to the present invention it's possible to have a system which allows to care for dental or orthopedic defects by optimum adaptation of the paste-like material to the implantation site and at the same time a controlled release of a active agent. Preferably the application method is minimal invasive even more preferable the application is by injection.

Thanks to the present invention, it is possible to treat various bone defects including large cavities in a new manner. In particular, large cavities could not or only under use of autogenous bone material be efficiently treated. However, due to the reliable and efficient osteoinductive and the osteoconductive properties of the paste of the present invention or the device which can be obtained by the method of the invention, treatment of bone defects which requires extensive bone augmentation or repair has now become possible without a second surgery.

The invention also encompasses the use of the paste of the invention or a paste which is obtainable by the method of the invention for the preparation of a pharmaceutical composition for bone augmentation.

The term "bone augmentation" refers to the induced formation of bone, which is indicated in order to treat bone defects, cavities in bones, or to treat diseases and disorders accompanied with loss of bone tissue or to prepare the subsequent setting of an implant. The diseases and disorders described in the following are well known in the art and are described in detail in standard medical text books such as Pschyrembel or Stedman.

Preferably, said bone augmentation follows traumatic, malignant or artificial defects.

Another embodiment of the present invention relates to the use of the paste of the invention or the preparation of a pharmaceutical composition for treating bone defects.

More preferably, said bone defects are long bone defects or bone defects following apicoectomy, extirpation of cysts or tumors, tooth extraction, or surgical removal of retained teeth.

The invention also relates to the use of the paste of the invention for filing of cavities and support guided tissue regeneration in periodontology.

Another embodiment of the present invention relates to the use of the paste of the invention for the preparation of a pharmaceutical composition for sinus floor elevation, augmentation of the atrophied maxillary and mandibulary ridge and stabilization of immediate implants.

Also within the scope of the present invention is a method for treating one or more of the diseases referred to in accordance with the uses of the present invention, wherein said method comprises at least the step of administering the paste of the invention in a pharmaceutically acceptable form to a subject. Preferably, said subject is a human.

Finally, the invention relates to a kit comprising the paste of the invention.

The parts of the kit of the invention can be packaged individually in vials or other appropriate means depending on the respective ingredient or in combination in suitable containers or multicontainer units. Manufacture of the kit follows preferably standard procedures which are known to the person skilled in the art.

### Contribution to the field by the present invention

Water insoluble polymer and filler together form the carrier/polymer composite paste material of the present invention, which binds an active agent to result in the paste of the present invention, which allows the sustained release of said active agent in vivo, once the paste is hardened within minutes to hours by diffusion out of the water soluble polymer (plasticizer) while aqueous body fluids diffuse into the polymer. In consequence the polymer solidifies due to its insolubility in water. (Shively, A.L. et al. 1995). The time until the full mechanical stability is reached can be controlled by the variation of the ingridents of the implant material.

In accordance with the present invention, the composite matrix is preferably based on a calcium phosphate which is beta tricalcium phosphate, alpha tricalcium phosphate, apatite or a calcium phosphate containing cement. Alternatively, said carrier is selected from the group consisting of calcium carbonate, magnesium carbonate, magnesium oxide or magnesium hydroxide and a polymer, preferably a biodegradable polymer. In such a composite carrier the calcium phosphate shows excellent local buffering capacity and the permeable composite structure avoids even local pH decrease when the polymer is degraded in vivo. Cytotoxic side effects due to degradation of the polymer are, hence, reduced or avoided. This is especially valid, since the ceramic carrier is chief ingredient of the ceramic carrier/polymer composite carrier material of the present invention, which preferably contains less than 40% of the polymer, most preferably less than 35% of PLGA even more preferably equal or less than 33 %of PLGA. In case of the PEG the ceramic carrier/polymer composite carrier material of the present invention preferably contains less than 60% of the water soluble polymer (PEG), most preferably less than 50% of PEG even more preferably equal or less than 45 % of PEG.

In case of the calcium phosphate the ceramic carrier/polymer composite carrier material of the present invention preferably contains less than 80% of the calcium phosphate, most preferably less than 60% of calcium phosphate even more preferably equal or less than 50 % calcium phosphate.

Furthermore, the ceramic/polymer composite paste of the present invention shows improved mechanical stability compared to conventional flowing systems using polymeric matrices for retarded release.

The ceramic/polymer composite carrier material of the present invention shows improved mechanical stability compared to conventional systems using polymeric matrices for retarded release. In porous embodiments the composite matrix allows for improved osteoconductive properties compared to prior art systems due to the porous system, in particular free of interconnected pores.

The ceramic/polymer composite carrier material of the present invention is suitable to replace conventional encapsualting polymeric granules important for retarded release. Due to the content of the ceramic carrier of the system (ceramic carrier plus polymer coating), the amount of polymer can be significantly reduced compared with totally polymer based conventional flowing systems using polymeric matrices. The reduced amount of polymer leads to a reduced risk of cytotoxicity. A further aspect of the invention is the increased mechanical stability of the composite material compared with totally polymer based scaffolds.

The invention will now be described by reference to the following examples which are merely illustrative and which shall not limit the scope of the present invention.

### Examples

### Example 1: Coating of β-TCP powder with rhGDF-5

333 mg beta-TCP and 147 µl rhGDF-5 in 10 mM HCl (3,4 mg/ml) are pipetted on the beta-TCP and absorbed. The damp powder is incubated for ca. 1 hour at 25°C and dried in a subsequent lyophilisation step.

### Example 2: Preparation of a rhGDF-5 / beta TCP biopolymer - plastisizer - paste

167µl PEG 400 and 83 mg PLGA are dissolved by gentle warming to get a viscous solution. After cooling at room temperature 333 mg coated rhGDF-5 beta TCP powder (see example 2) was added under continuous stirring to get a homogenous paste.

### Example 3: Preparation of the placebo beta TCP - biopolymer - plasticizer - paste

1 ml PEG 400 and 500 mg PLGA are dissolved by gentle warming to get a viscous solution. After cooling at room temperature the 2000 mg beta TCP powder was added under continuous stirring to get a homogenous paste.

### Example 4: Extraction of the immobilised protein

### (step A)

Extraction of 417 mg hardened test sample (1h at 4 °C) in 1 ml chloroform solution to dissolve the polymer. After removing the chloroform-polymer solution the residual chloroform was removed in an desiccator under vacuum.

### (step B)

For the Extraction of the immobilised protein from the beta TCP the resulting sample from step A (coated and polymer free granules) were immersed in 8M Urea, 10mM Tris, 100mM EDTA for 1 h at 4 °C.
In the subsequent step the solution was centrifuged (9300* g, 3 min). The protein content and the degradation products in the supernatant were quantified by RP-HPLC (example 4).

### Example 5: Preparation of test samples

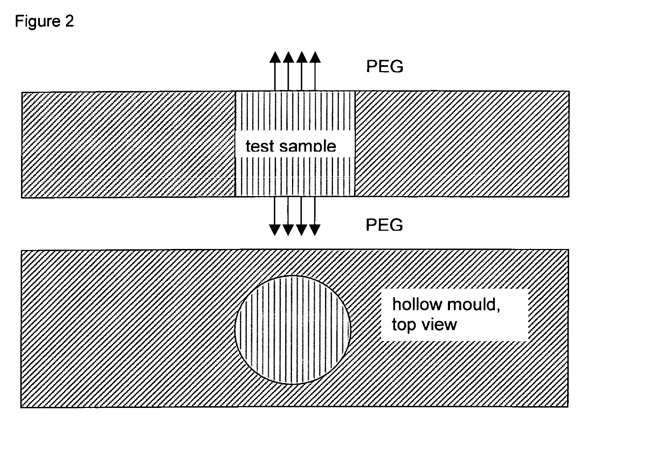

The hardening of the test samples takes place by replacement of the PEG with water from the body fluid. Under simulated physiological conditions the body fluid was represented by phosphate buffered saline (PBS). After casting the paste into a hollow mould subsequently the paste was hardend by incubating the hollow mould in PBS during gentle agitation of the PBS to support the diffusion of PEG away from the test sample (see figure 2). The final hardness was reached already after three hours at room temperature.

### Example 6: Quantification and determination of chemical modifications of the extracted protein:

The amount of chemical modifications i.e. oxidation of bone growth factor in solutions containing extracted protein was determined by RP-HPLC. The sample is applied to a Vydac C18 column (2.1 x 250 mm) which has been equilibrated with 0.15 % TFA, 20 % acetonitrile. The elution takes place with 0,15% TFA, and a stepwise gradient of 20 % - 84 % acetonitrile (flow: 0.3 ml/min). The elution is observed by measuring the absorption at 220 and 280 nm. The quantification takes place via the ratio peak area of modified species in relation to the total peak area.

### Example 7: Determination of the E module according DIN ISO 604 2002

The modulus of elasticity was determined with a conventional material testing machine.

### Example 8: Improved mechanical properties by variation of beta -TCP-grain size Different grain sizes were used:

- beta-TCP approximately 10 - 100 µm
- beta-TCP approximately 100 - 300 µm
- beta-TCP approximately 10 µm

### Example 8: Improved mechanical properties by variation of the polymer content

The different pastes are prepared by variation of the method described in example 3. To manufacture test samples the paste was hardened according example 5. The mechanical stability war determined as described in example 9.

**Table 1:**

| composition of the paste: | | |
|---|---|---|
| 14 % PLGA | 29 % PEG | 57 % beta TCP |
| 18 % PLGA | 29 % PEG | 53 % beta TCP |
| 21 % PLGA | 29 % PEG | 50 % beta TCP |
| 25 % PLGA | 29 % PEG | 46 % beta TCP |

**Table 2:**

| composition of hardened test samples: | |
|---|---|
| 20 % % PLGA | 80 % beta TCP |
| 25 % PLGA | 75 % beta TCP |
| 30 % PLGA | 60 % beta TCP |
| 35 % PLGA | 55 % beta TCP |

**Table 3:**

| Measured E-modules | |
|---|---|
| | E-Modul [MPa] |
| 25% PLGA | 47,7 |
| 30% PLGA | 87,2 |
| 35% PLGA | 113,2 |

### Example 9: Improved mechanical properties by variation of the polymer content

Different PLGA qualities were used to manufacture the test bodies

| **PLGA type** | **molecular weight [g/mol]** |
|---|---|
| 20% PLGA (Resomer® RG 503 H) | 18600 |
| 20 % PLGA (Resomer® RG 502 H) | 34000 |

| | E-Modul [MPa] |
|---|---|
| 20% PLGA (Resomer® RG 503 H) | 47,7 |
| 20 % PLGA (Resomer® RG 502 H) | 53,3 |

### Example 10: Determination of the pore size and pore orientation

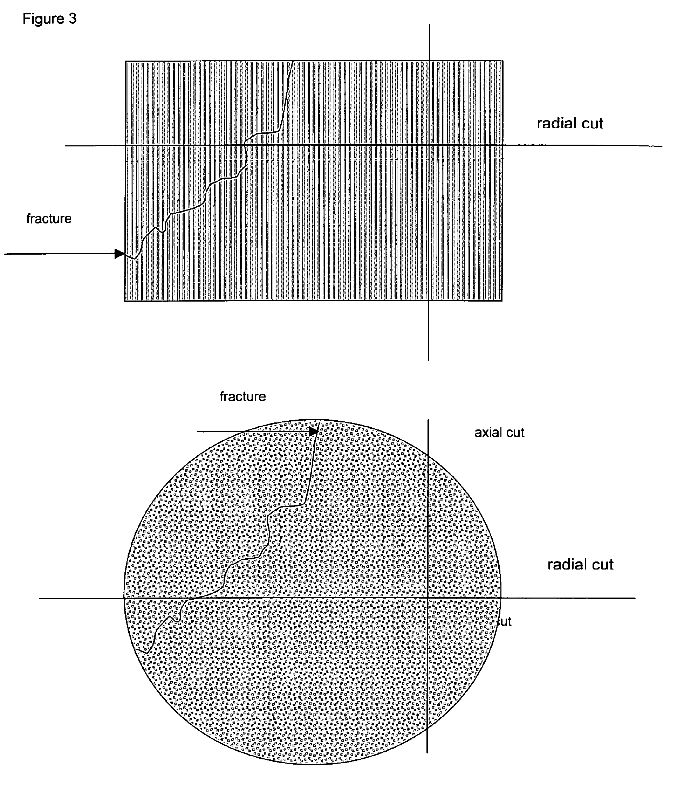

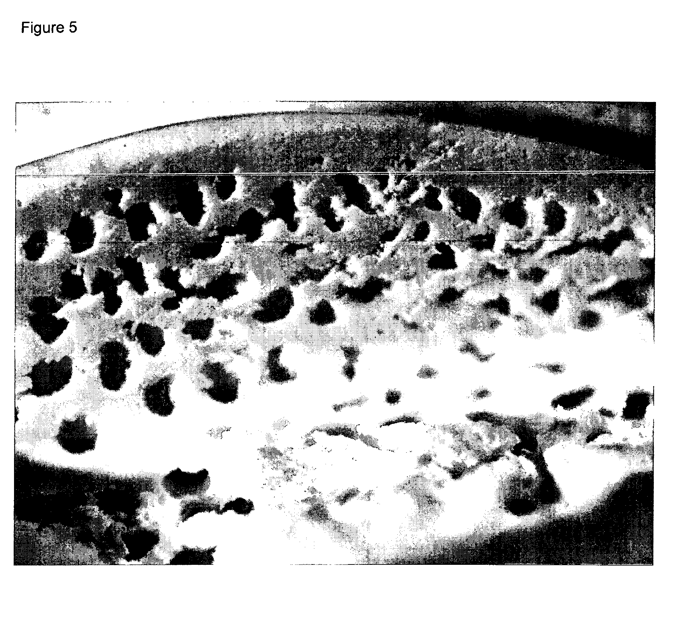

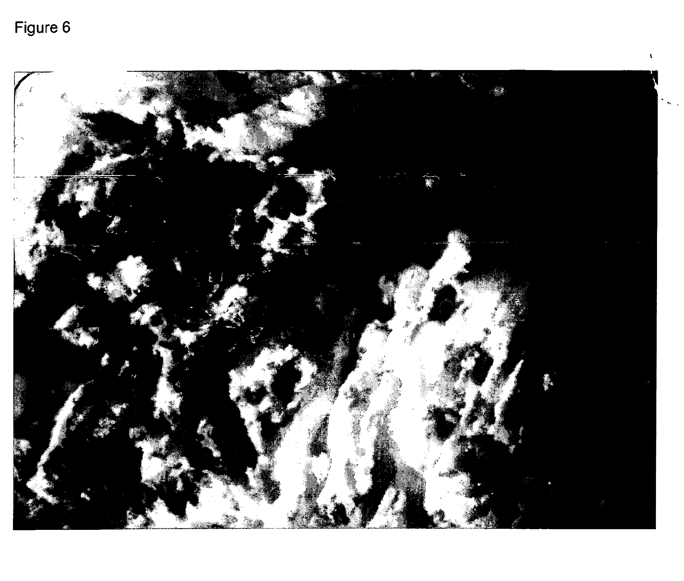

During hardening of the test samples according example 5, the structured porosity is formed. The pore size and the pore orientation was determined by the composition of the paste by the content of the water soluble plasticizer. Various formulations are tested and analysed. The pore size and structure were analysed by light microscopy an by raster electron microscopy of various cross sections through the test samples (figure 3). The structured interconnecting pores are formed in the direction to surrounding tissue (figure 5 and 6). The pore size was determined form 50 - 200 nm. The structured pores enable a leaded cell rapid movement into the core of the scaffold to support the replacement and accelerated vasculatisation with new tissue.

References:
Agrawal, C. M. et al. (1997) "Technique to Control pH in Vicinity of Biodegrading PLA-PGA Implants"; J. Biomed. Mater. Res. (Appl. Biomater.), 38: 105-114
Breitenbach Jörg, (2002) "Melt extrusion: from process to drug delivery technology", Eur. J. Pharm. Biopharm. 54, 107-117
Celeste A.J., et al. (1990) "Identification of transforming growth factor ..."; Proc. Natl. Acad. Sci. U.S.A. 87:9843-9847.
Chang, S. et al. (1994); "Cartilage-derived morphogenetic proteins..."; J. Biol. Chem. 269: 28227-28234.
Draenert, K. et al (2001), Trauma Berufskrankh. 3, 293 - 300.
Driessens, F.C.M. et al. (2002) "The Ca/P range of nanoapatitic calcium phosphate cements" Biomaterials 23, 4011-4017
Dunn et al. US patent 5,702,716
Durucan, C. et al.; "Calcium-deficient hydroxyapatite-PLGA composites: mechanical and microstructural investigation." J. Biomed. Mater. Res. 51:726-724.
EMEA, ICH Topic Q 3 C, Impurities: Residual Solvents
Friess, W. et al. (1998); Pharm. Dev. Technol. 4, 387 - 396.
Friess, W. (1999); "Collagen - biomaterial for drug delivery"; Eur J Pharm Biopharm 45: 113-36.
Gao, T et al. (1996); Int. Orthopaedics 20, 321 - 325.
Gombotz, W et al. (1996) in Formulation, characterization and stability of protein drugs, Plenum Press, New York, USA, pp 219 - 245.
Griffith, D.L. et al. (1996); "Three-dimensional structure of recombinant human..."; Proc. Nati. Acad. Sci. U.S.A. 93: 878-883.
Herbert, P. et al., 1998. A large scale process to produce microencapsulated Proteins. Pharm Res., 15: 357-361
   Hoetten, G. et al. (1994); "Coning and expression of recombinant human growth/differentiation factor 5."; Biochem. Biophys. Res. Commun. 204: 646-652.
J.O. Hollinger et al. (1996); "Poly(α-hydroxy acids): carriers for bone morphogenetic proteins, Biomaterials"; 17: 187-194
Hotz, G et al. (1994); Int. J. Oral Maxillofac. Surg. 23, 413 - 417.
Katagiri, T. et al. (1990); Biochem. Biophys. Res. Commun. 172: 295-299.
Kenley, R.A., et al. (1993) Pharm. Res. , 10 (10) 1393-1401.
Lind, M et al. (1996); J. Orthopaedic Res. 14, 343 - 350
Lind, M. (1996); Acta. Orthop. Scand. 67: 407-17.
Middleton, J.C. et al. (2000); "Synthetic biopolymers as orthopedic devices"; Biomaterials 21: 2335-2346
Nishitoh, H. et al (1996); J Biol. Chem. 271: 21345-21352.
Oezkayanak, E. et al. (1990); "OP-1 cDNA encodes an osteogenic protein in the TGF-beta family"; EMBO J. 9: 2085-2093
Rueger, J.M. et al (1996); "Knochenersatzmittel"; Unfallchir. 99: 228-236
Scheufler, C. et al. (1990); "Crystal structure of human bone morphogenetic protein-2 at 2.7 A resolution".
Schiller, C. et al. (2003); "Carbonated calcium phosphates are suitable pH-stabilising fillers for biodegradable polyesters"; Biomaterials 24:2037-2043.
Schmidmaier, G. et al. (2000); "Local liberation of IGF-I and TGF-beta 1 from a biodegradable poly(D,L-lactide) coating of implants accelerates fracture healing" Chirurg 71: 1016-1022
Schmitt, J. et al. (1999); J. Orthop. Res. 17: 269-278.
Seeherman, H. (2003), "A review of preclinical program development for evaluating in injectable carriers for osteogenic factors" J. Bone. Joint. Surg. Am. 85-A Suppl. 3, 96-108
Shively M.L. et al. (1995), "Physico-chemical characterization of a polymeric injectable implant delivery system" J. Controlled Rel. 33, 237-243
Shore, E.M. et al. (1997); "Human bone morphogenetic protein-2 (BMP-2) genomic DNA sequence".
Storm & Kingsley (1999); Development Biology, 209, 11-27.
Terheyden, H et al. (1997); Mund Kiefer Gesichtschir. 1, 272 - 275.
Tormälä, P et al. (1995) "Biodegradable polymers in orthopaedics: experimental and clinical" Mat. Clin. Appl. 639-651
Tormälä, P et al. (1992) "Biodegradable self-reinforced composite materials; manufacturing structure and mechanical properties" Clin. Mater. 10, 29-34
Vert, M. (1989); "Bioresorbable polymers for temporary therapeutic applications" Angew. Makromol. Chem. 166/167: 155-168
Wang, E.A. et al. (1990); "Identification of transforming growth factor beta family members present in bone-inductive protein purified from bovine bone."; Proc. Natl. Acad. Sci. U.S.A. 87: 9843-9847.
Wintermantel, E. et al., Medizintechnik mit biokompatiblen Werkstoffen und Verfahren, S. 7, 3. Auflage, Springer 2002, ISBN 3-540-41261-1
Wozney, J.M. et al. (1998); Clin. Orthop. 346: 26-37.
Wozney, J.M. et al. (1988); Science 242: 1528-1534.

## Claims

**1.** A method for the production of an in situ forming paste comprising the steps of:
(a) providing solution comprising a plasticizer, which is a water soluble polymer, and a water insoluble polymer to get a viscous liquid;
(b) dissolving an active agent in said viscous liquid; and
(c) mixing said viscous liquid consisting of the water insoluble polymer and the plasticizer with the water insoluble solid filler to prepare a paste

**2.** A method for the production of an in situ forming paste comprising the steps of:
(a) providing solution comprising a plasticizer, which is a water soluble polymer, and a water insoluble polymer to get a viscous liquid; and
(b) mixing said viscous liquid consisting of the water insoluble polymer and the plasticizer with a water insoluble solid filler to prepare a paste, wherein said filler comprises an active agent which is coated on said solid filler

**3.** The method of claim 1 or 2, optionally comprising the step of adding a water soluble filler for pore building.

**4.** The method of any one of claims 1 to 4, wherein said in situ forming paste has osteoinductive and osteoconductive or cartilage regenerating properties in vivo.

**5.** The method of any one of claims 1 to 4, wherein said water insoluble, said water soluble polymer and said water insoluble solid filler are biocompatible, biodegradable and/or bioresorbable.

**6.** The method of any one of claims 1 to 4, wherein said biocompatible, biodegradable and/or bioresorbable water insoluble polymer is selected from poly(α-hydroxy acids), poly (ortho esters), poly(anhydrides), poly(aminoacids), polyglycolid acid (PGA), polylactid (PLLA), PDLLA, PLGA, poly(3-hydroxybutyricacid) (P(3-HB)), poly(3-hydroxy valeric acid) P(3-HV), poly(p-dioxanone) (PDS), poly(ε-caprolactone) (PCL), polyanhydride (PA) polyorthoester, polyethylene (PE), polypropylene (PP), polyethylenerephtalate (PET), polyglactine, polyamide (PA), polymethylmethacrylate (PMMA), polyhydroxymethylmethacrylate (PHEMA), polyvinylchloride (PVC), polyvinylalcohole (PVA), polyetrafluorethylene (PTFE), polyetheretherketone (PEEK), polysulfon (PSU), polyethyleneglycol (PEG), polyvinylpyrrolidone, polyurethane or polysiloxane.

**7.** The method of any one of claims 1 to 4, wherein said plasticizer is selected from the group consisting of polyethylene glycol 400, PEG 200, PEG 300, PEG 600, 1,3 butandiole, castor oil, N-methyl-2-pyrrolidone, 2-pyrrolidone, C2 to C6 alkanols, propylene glycol, solketal, acetone, methyl acetate, ethyl acetate, ethyl lactate, methyl ethyl ketone, dimethylformamide, dimethyl sulfoxide, dimethyl sulfone, tetrahydrofuran, caprolactam, decylmethylsulfoxide, oleic acid, propylene carbonate, triacetin, N,N-diethyl-m-toluamide, or I-dodecylazacycloheptan-2-one.

**8.** The method of any one of claims 1 to 4, wherein said water insoluble solid filler is selected from calcium phosphate, chitosan, collagen, alginate, calcium sulfate, cacium carbonate, magnesiumoxide, magnesium hydroxide, magnesium carbonate, silicium dioxide, poly(2-hydroxyethyl methacrylate), hyaluronic acid and derivatives, cellulose and derivatives, starch and derivatives and / or any combinations thereof.

**9.** The method of any one of claims 1 to 4, wherein said water insoluble solid filler is beta tricalcium phosphate, alpha tricalcium phosphate, apatite or a calcium phosphate containing cement.

**10.** The method of claim 3, wherein said water soluble filler is selected from the group consisting of polyethyleneglycol 4000, sucrose, mannitol, sodium chloride, solid physiological tolerable and soluble, non toxic substances like trehalose, sorbitol physiological amino acids e.g. glycine, glutamin, arginine, sodium citrate, sodium succinate, sodium phosphates.

**11.** The method of any one of claims 1 to 10, wherein said active agent is selected from the group consisting of hormones, cytokines, growth factors, antibiotics and small molecules.

**12.** The method of any one of claims 1 to 10, wherein said active agent is parathyroid hormone (PTH) and/or PTH 1-34 peptide.

**13.** The method of any one of claims 1 to 10, wherein said active agent is an osteoinductive or cartilage inductive protein.

**14.** The method of claim 12, wherein said osteoinductive protein is a member of the TGF-β family is a member of the BMP subfamily.

**15.** The method of claim 14, wherein said member of the BMP family is selected from BMP-1, BMP2, BMP-3, BMP-4, BMP-5, BMP-6, BMP7, BMP-8, BMP-9, BMP-10, BMP-11, BMP-12, BMP-13, BMP-14, BMP-15 and BMP-16.

**16.** The method of claim 14, wherein said member of the TGF-β family is selected from the group consisting of GDF-1, GDF-2, GDF-3, GDF-4, GDF-5, GDF-6, GDF-7, GDF-8, GDF-9, GDF-10 and GDF-11.

**17.** The method of claim 13, wherein said cartilage regenerating protein is MIA.

**18.** The method of any one of claims 1 to 17, wherein said in situ forming paste is free of toxic substances.

**19.** The method of any one of claims 1 to 17, wherein said in situ forming paste is free of infectious material.

**20.** An in situ forming paste which is obtainable by the method of any one of claims 1 to 19.

**21.** A pharmaceutical composition comprising the in situ forming paste of claim 20.

**22.** Use of the in situ forming scaffold of claim 20 for the preparation of a pharmaceutical composition to be used for bone augmentation. 23. The use of claim 22, wherein said bone augmentation follows traumatic, malignant or artificial defects or is a prerequisite for the subsequent setting of an implant.

**24.** Use of the in situ forming scaffold of claim 20 for the preparation of a pharmaceutical composition for treating bone defects.

**25.** The use of claim 24, wherein said bone defects are long bone defects, defects in the maxillofacial area or bone defects following apicoectomy, extirpation of cysts or tumors, tooth extraction, or surgical removal of retained teeth.

**26.** Use of the in situ forming scaffold of claim 20 for the preparation of a pharmaceutical composition for treating degenerative, traumatic disc disease and spinal fusion.

**27.** Use of the in situ forming scaffold of claim 20 for the preparation of a pharmaceutical composition for treating bone dehiscence.

**28.** Use of the in situ forming scaffold of claim 20 for the preparation of a pharmaceutical composition to be used for sinus floor elevation or augmentation of the atrophied maxillary or mandibular ridge.

**29.** Use of the in situ forming scaffold of claim 20 for the preparation of a pharmaceutical composition for filling cavities and/or support guided tissue regeneration in periodontology.

**30.** Use of the in cartilage regenerating paste of claim 20 for the preparation of a pharmaceutical composition for promoting chondrogenesis.

**31.** Use of the cartilage regenerating paste of claim 20 for the preparation of a pharmaceutical composition to be used for the treatment of at least one bone disease.

**32.** The use of claim 31, wherein said bone disease is selected from the following diseases in which chondrogenic differentiation is involved: osteoarthritis; rheumatoid arthritis, injury of articular cartilage due to trauma, maintenance of chondrocyte phenotypes in autologous chondrocyte transplantation, reconstruction of cartilage in the ear, trachea or nose, osteochondritis dissecans, regeneration of intervetebral disk or meniscus, bone fracture and/or osteogenesis from cartilage.

**33.** A kit comprising the in situ forming paste of claim 20.
